(19)

Europäisches
Patentamt
European
Patent Office
Office européen
des brevets

(11) **EP 3 881 793 A1**

(12) **EUROPEAN PATENT APPLICATION**

(43) Date of publication:
22.09.2021 Bulletin 2021/38

(51) Int Cl.:
*A61B 90/00* (2016.01)   *G06N 3/02* (2006.01)

(21) Application number: 20305281.6

(22) Date of filing: 17.03.2020

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **CHU de NICE**
**06200 Nice (FR)**

(72) Inventors:
• SEJOR, Eric
  06000 NICE (FR)
• MUSSE, Olivier
  35000 RENNES (FR)

(74) Representative: **Marks & Clerk France**
**Immeuble "Visium"**
**22, avenue Aristide Briand**
**94117 Arcueil Cedex (FR)**

(54) **SURGICAL INSTRUMENT AND COMPUTER-IMPLEMENTED METHOD FOR DETERMINING THE POSITION AND ORIENTATION OF SUCH SURGICAL INSTRUMENT**

(57)   The invention relates to a surgical instrument having a tip end and comprising at least one marker configured for identifying the surgical instrument, indicating the position of the at least one marker and the orientation of the surgical instrument. The present invention also relates to a computer-implemented for determining the position and orientation of the at least one tip end of the at least one surgical instrument comprising at least one marker and especially performing a zoom on a computed area of interest formed by the at least one tip end of the at least one surgical instrument.

[Fig. 1]

**Description**

FIELD OF THE INVENTION

The invention is in the field of surgical instruments having a tip end and markers configured for identifying the surgical instruments. More particularly, the present invention relates to a computer-implemented method for determining the position and orientation of at least one tip end of at least one surgical instrument comprising at least one marker and especially performing a zoom on a computed area of interest formed by the at least one tip end of the at least one surgical instrument.

BACKGROUND ART

During surgery, practitioners manipulate, directly or through robots, several surgical instruments that allow them to manipulate different tissues and to perform surgical procedures. The surgical instruments are adapted to be efficient in limited spaces. In particular, medical endoscopy techniques and minimally invasive surgical techniques such as laparoscopy and thoracoscopy involve the use of particularly fine surgical instruments, generally elongated in shape to occupy a minimum of volume.

Also, it is necessary for practitioners during such procedures to have a clear view of the environment in which the instruments are used. Cameras may therefore be used, especially during surgical procedures when it is not possible to see the operation area with the naked eye or when the vision is no longer enough to allow a safe operation. Such cameras can be arranged in different ways: for instance, outside the patient or inserted into the patient. Using a camera allows obtaining an image usually displayed on a screen so as for the practitioner to see the manipulations more closely.

Certain surgical procedures comprise multiple steps in different locations which require replacing the camera multiple times so as to direct the view angle of the camera on different specific surgical field of interest for each step. Also, as the camera must be replaced by an assistant, the focus must be updated to ensure the quality of the image displayed to the practitioner.

However, moving the camera is a constraint as, in the case the camera is inserted into the patient, it requires frequent manipulation which should be avoided due to the potential consequences over the surgical procedure.

Furthermore, moving the camera requires time so as to place such camera with the right angle of view and the right distance and obtaining a good focus on the surgical field of interest. Any loss of time during a surgical procedure is not wished and may have consequences over the patient health, the duration of anesthesia, the attendance time of the health care team and the infectious risk.

Worse, the camera or the patient may also be moved unintentionally during the surgical act according to different manipulation in relation or not with the surgery act. Such incident would require replacing the camera so as for the camera to be directed to the specific surgical field where a surgical act will be performed.

There is therefore a need for a method and/or instruments which allow displaying to the practitioner an image of a specific surgical field of interest where a surgical act is to be performed, while overcoming the need of replacing a camera and await the focus of such camera.

SUMMARY OF THE INVENTION

To achieve the foregoing object, a surgical instrument, computer-implemented method, learning database and system as further described in the appended independent claims 1, 7, 14 and 15 are provided.

To this effect, the invention discloses a surgical instrument having a tip end and comprising at least one marker, wherein said at least one marker is configured for identifying the surgical instrument, for indicating the position of said at least one marker on the surgical instrument and for indicating at least the orientation of the tip end of the surgical instrument.

By "surgical instrument", it is understood any instrument which is adapted for carrying out any step of a surgical procedure. A surgical instrument may be any surgical instrument among aspiration, bipolar forceps, clamp, cannula, carrier bag, cautery hook, clip applier, curved grasper, Endoloop, fenestrated grasper, five retractor, long curved grasper, long flat grasper, linear or circular stapler, long grasper, measurement devices, needle, needle driver, robotic cautery hook, robotic fenestrated grasper, robotic needle driver, robotic vessel sealer, retractor, short curved grasper, short flat grasper, scissors, trocar, ultrasonic scissors, Veress needle. Generally but without limiting the present invention to particular surgical instruments, the surgical instruments have an elongated shape between a proximal end, which corresponds to a manipulating end for a practitioner or a robot, and a distal end, which corresponds to an operational end which is expected to interact with an object or a tissue.

By "tip end", it is understood at least one point, or a line, or a surface, or a volume of the surgical instrument which corresponds to the surgery interaction point or line or surface or volume of the surgical instrument. It may be referred as the distal end, being on the opposite side of the proximal end of the surgical instrument. For instance, when the surgical instrument is a needle, the tip end corresponds to a point of the surgical instrument which is adapted to interact with the patient tissue during surgery. When the surgical instrument comprises multiple parts at its tip end which are adapted to interact with the patient tissue, i.e. surgical staplers or graspers or scissors, the tip end may correspond to a virtual point being at the center of the multiple parts, or a virtual line between the multiple parts, or a virtual surface between the multiple parts, or a virtual volume formed by the multiple parts at the tip end or the surgical instrument. Also, the tip end of a surgical instrument may correspond to the virtual surface or the virtual volume which is the bounding box of all or only some of the multiple parts of the surgical instrument. Such bounding box may also comprise margins. For instance, in the case of surgical scissors, the tip end may correspond to the virtual line between the blades which correspond to the cutting line or may correspond to a virtual point at the center between the distal extremities of the scissors blades. The man of the art is able to determine what corresponds to the tip end of any surgical instrument depending on the shape and the interaction zone of the surgical instrument with the patient tissue or any object.

By "said at least one marker is configured for identifying the surgical instrument", it is understood that the at least one marker comprises information means configured for identifying the surgical instrument.

By "identifying the surgical instrument", it is understood that the said at least one marker of the surgical instrument comprises information means which allow determining at least one characteristic of the surgical instrument such as a group number, which group number may correspond to a particular hierarchy rank according to a parameter (i.e. importance, dangerousness, priority in the surgical procedure). The at least one characteristic of the surgical instrument may also comprise the type of surgical instrument, and/or the type of tip end of the surgical instrument.

By "the position of said at least one marker on the surgical instrument", it is understood the position of the center of the at least one marker on the surgical instrument, or the position of each ends of the at least one marker on the surgical instrument. In the case of multiple markers, it corresponds to the center of each marker or the position of each ends of the at least one marker on the surgical instrument. The position may also correspond to the spatial position of the at least one marker on the surgical instrument, which corresponds to the distance between each marker and the tip of the surgical instrument.

By "the orientation of the tip end of the surgical instrument", it is understood that the at least one marker comprises information means for indicating which side of the at least one marker is the closest to the tip end, e.g. the distal end which is expected to interact with the patient tissue or any object.

According to a first embodiment of the present invention, there is provided a surgical instrument as further described in the appended independent claim 1.

Further embodiments are described in the appended dependent claims.

Advantageously, the said at least one marker of the surgical instrument comprises a guide part and a code part, the guide part being configured to locate a marker, and wherein the guide part is of at least one color and the code part being of at least one other color which is different from the at least one color of the guide part.

The guide part corresponds to a part of the marker which allows locating a marker, for instance within a frame captured by a camera. The guide part is particularly configured for being noticeable within a frame among the other elements captured with a camera. The guide part allows locating a marker and especially the code part of the marker. The guide part has a pattern which may be of various forms with different thicknesses and may comprise dots, lines, characters, or any other form which is visible to the naked eye and/or by a camera, the pattern being on the surface of the surgical instrument.

The code part corresponds to a part of the marker which allows obtaining information for identifying the surgical instrument and/or the position of the marker and/or orientation of the marker. The code part pattern on the surface of the surgical instrument may be of various forms with different thicknesses so as to be recognizable and identifiable. The code part may comprise dots, lines, characters, or any other form which is visible to the naked eye and/or by a camera.

The guide part may surround the code part, meaning that the guide part is placed at least on one side of a marker, a marker having two sides, one side being closer to the tip end of the surgical instrument, e.g. the distal end expected to interact with the patient tissue, and the other side being closer to the proximal end of the surgical instrument.

The combination of a guide part and a code part of at least one marker on the surgical instrument allows firstly locating the marker by means of the guide part which is particularly noticeable. Locating the guide part of the at least one marker of the surgical instrument allows secondly locating the code part of the at least one marker. Then the code part allows identifying the at least one marker and therefore the surgical instrument. The code part also allows indicating the position of said at least one marker on the surgical instrument and indicating at least the orientation of the tip end of the surgical instrument.

The guide part of the at least one marker may be of at least one color and the code part may be of at least one color which is different from the at least one color of the guide part. Such difference of color allows distinguishing the guide part from the code part so as to clear out what is seen from the at least one marker. Moreover, in the case a part of the marker cannot be seen, for instance because it is hidden behind any tissue or because it is behind another surgical instrument, the difference of color may provide information on which part of the marker can be seen.

Advantageously, the guide part of the at least one marker of the surgical instrument is made of a contrasting color. By "contrasting color" it is understood that the guide part is noticeable within a frame and among the colors of the environment in which the at least one marker is located. The environment where the at least one marker and the guide part of that at least one marker are seen may comprise a majority of colors or hues corresponding to human or animal tissues, preferably internal tissues. For instance, the colors present in the human body are mostly blue, purple, or red (blood, veins, and arteries), orange or yellow (fat) and may be lightened or darkened according to the luminosity.

Advantageously, the code part of the at least one marker of the surgical instrument includes a plurality of circumferential bands. By "bands", it is understood that the code part may comprise a plurality of lines of different thicknesses and different colors being alternating. In the case of two colors being black and white, it may be understood that there are only black bands spaced by white bands or that there is an alternate pattern of black and white bands. The code part is similar to a barcode, encoding information about the position and the orientation of the surgical instrument. A pattern of multiple bands corresponds to information means as the multiple bands may for example be converted to a binary code or any other code. By "circumferential bands", it is understood that the surgical instrument, without limiting the invention to such aspect, has generally an elongated shape along an axis, and that the circumferential bands are disposed orthogonally to the axis of the surgical instrument. Also, a circumferential band may be understood as a band on a radial cross section of the surgical instrument, i.e. a strip around the axis section. Such circumferential shape of the bands allows the code part to be visible to a camera or the naked eye even though the surgical instrument rotates. The guide part may also correspond to circumferential bands.

Advantageously, the at least one marker of the surgical instrument has a circumferential shape. By "circumferential shape", it is understood that the surgical instrument, without limiting the invention to such aspect, has generally an elongated shape along an axis, and that the marker of circumferential shape is disposed orthogonally to the axis of the surgical instrument. Also, a circumferential shape of the marker may be understood as a marker on a radial cross section of the surgical instrument, i.e. a marker around the axis section. Such circumferential shape of the marker allows the marker to be visible to a camera or the naked eye even though the surgical instrument rotates.

Advantageously, the surgical instrument further comprises a manipulating section at a first end, e.g. the proximal end, a tool section at a second end, e.g. the distal end, and an axis section between the manipulating section and the tool section, and wherein said at least one marker is located on the axis section. Hence it is possible to replace the manipulating section or the tool section by another manipulating section or tool section respectively so as to use only one axis section for multiple surgical instruments or only for replacing the tool section.

The surgical instrument of the present invention may comprise multiple markers so as to avoid the possibility of an occlusion of one or more markers of a surgical instrument at once. For instance, when any surgical instrument is moved, a part of the surgical instrument may be hidden behind a tissue or an object such as another surgical instrument.

The invention also discloses a computer-implemented method for determining the position and the orientation of the at least one tip end of a surgical instrument, the surgical instrument comprising at least one marker according to anyone of the previous paragraphs, the method comprising the steps of:

-    receiving at least one frame captured by a camera;

-    detecting within the at least one frame received at least one marker;

-    identifying said at least one marker of a surgical instrument from the detected at least one marker so as to obtain information of said at least one marker; and

-    determining the position and orientation of the at least one tip end of the surgical instrument using information of said at least one marker.

The computer-implemented method of the invention allows obtaining, from at least one frame captured by a camera, information on the identity of the surgical instrument, determining the position of the at least one marker and therefore, from the information obtained of the at least one marker, determining the position and orientation of the tip end of the identified surgical instrument. The determined position and orientation of the tip end of the identified surgical instrument allow verifying the right positioning of the surgical instruments regarding any object to manipulate so as to improve the precision of the actions performed by the practitioner and/or a surgical robot.

By "detecting within the at least one frame received at least one marker", it is understood that the method allows obtaining from the at least one frame received a list of the at least one marker identifiable in the frame.

The "information of the at least one marker" used corresponds to at least one characteristic of the said marker such as an identification number of the surgical instrument, which identification number may correspond to a particular hierarchy rank according to a parameter (importance, dangerousness, priority in the surgical procedure). The at least one characteristic of the surgical instrument may also comprise the type of surgical instrument, the type and number of tip ends of the surgical instrument. The "information of the at least one marker used" may also correspond to an identification number of the marker. The identification number of the marker may correspond for instance to the position and orientation of the marker on the surgical instrument.

According to a first embodiment of the present invention, there is provided a computer-implemented method as further described in the appended independent claim 7.

Further embodiments are described in the appended dependent claims.

Advantageously, the computer-implemented method further comprises the steps of:

- computing an area of interest according to the determined position and/or orientation of the at least one tip end; and

- performing a zoom on the computed area of interest.

By "an area of interest", it is understood that the tip end of the surgical instrument or the tip ends of the at least one surgical instrument form an area in which these tip ends may interact with an object or between each other. The area of interest corresponds to a part of the frame captured by the camera which comprises at least the end tip of a surgical instrument. The area may comprise the tip ends of a single surgical instrument or multiple surgical instruments and may be rectangular, round or may have a singular shape. In particular, in case of multiple instruments having different hierarchy ranks, the area of interest may correspond to the bounding box of the tip ends of the surgical instruments which have one specific hierarchy rank, for example the highest or the lowest. For instance, in case of surgical graspers of hierarchy rank 2 and surgical scissors of hierarchy rank 1, the area of interest may be defined as being the area of interest corresponding to the bounding box of the tip ends of the surgical instruments of hierarchy rank 1, i.e. an area with margins around the virtual cutting line of the surgical scissors.

The skilled person in the art is able to define according to the surgical instruments what margin is necessary for the area of interest, which depends mostly on the function of the at least one surgical instrument, the end tips of the at least one surgical instrument and on the predictable tremor or vibration of the use of the at least one surgical instrument. For instance, the area of interest of only one surgical instrument being a needle which has only one tip end may be of a rectangular shape with the tip end being at the center, the margin being of for instance few centimeters around the tip end of the needle, i.e. 4 centimeters.

Preferably, the computed area of interest corresponds to the bounding rectangle of the at least one tip end of at least one surgical instrument. Such bounding rectangle may also comprise vertical and horizontal margins according to the set of surgical instruments used. The computed area of interest may take into account the output screen aspect ratio.

By "performing a zoom on the computed area of interest", it is understood that the computer-implemented method enables displaying to the practitioner the computed area of interest on a screen or any device which the practitioner may use during a procedure. For instance, the computed area of interest may be displayed on only a part of a screen as a magnifying glass.

Such a zoom on the computed area of interest allows obtaining an enlarged image of an area of interest. By such zoom, the practitioner may obtain more information on the area of interest as details which could not be seen by the naked eye may appear clearer to the practitioner view. Obtaining more information for a procedure results in a better preparation of the next acts and a better prediction of the next steps. Also, obtaining a zoom on an area of interest allows a better estimation of the extent of the tools movement. The computer-implemented method of the present invention allows ensuring the accuracy and the success of a procedure by providing more reliable information while being faster as no manipulation of a camera is necessary.

In order to reduce the update of the area of interest according to every new frame, and therefore an update of the zoom on the computed area of interest for every new frame which would be uncomfortable to watch, the computer-implemented method of the present invention may be followed by an algorithm to smooth the updates of the computed area of interest.

Advantageously, the step of detecting at least one marker in the computer-implemented method comprises:

- segmenting by colorimetry the at least one frame received;

- determining at least one part of a marker in the segmented frame; and

- detecting at least one marker associated to at least one determined part.

By "segmenting by colorimetry", it is understood that it is extracted from the at least one frame received a list of the guide parts identifiable, which may correspond to connected components which meet colorimetry thresholds. The connected components may correspond to groups of neighboring pixels. For instance, in a group of 8 pixels being centered around a pixel, if all the pixels meet the threshold of being of a contrasting color, e.g. green, they may form a connected component which corresponds to the guide part of a marker on a surgical instrument.

By "determining at least one part of a marker in the segmented frame", it is understood that it is provided a list of the connected components which may be determined in the frame segmented by colorimetry.

By "associated to at least one determined part", it is understood that the parts of marker determined in the segmented frame are considered as being a unique object being one marker. For instance, when a marker comprises two guide parts on a surgical instrument and they both appear onto the segmented frame, the computer-implemented method of the invention allows associating the two guide parts and the content between them on the segmented frame and associate these parts to a unique marker. The content between the two guide parts may correspond to the code part of a marker on the surgical instrument. When a marker comprises only a code part or when the guide part and the code part are confounded, the computer-implemented method of the invention allows associating the only part to a unique marker.

The step of detecting at least one marker comprising segmenting by colorimetry the at least one frame received; determining at least one part of a marker in the segmented frame; and detecting at least one marker associated to at least one determined parts allows improving the robustness of the computer-implemented method of the invention. Such step also allows ensuring the correct detection of the at least one marker by improving the specificity and sensitivity of the method.

Preferably, only the connected components with connectivity 8 in the frames are extracted. From all the connected components extracted, the surface of each connected component is computed and all of the connected components outside low and high surface thresholds are removed.

Advantageously, the step of identifying said at least one marker of a surgical instrument of the computer-implemented method comprises:

- extracting at least one part of the captured frame corresponding at least to the code part of said at least one detected marker and segmenting said at least one extracted part;

- recognizing said at least one marker from the segmented at least one extracted part; and

- identifying said at least one marker of a surgical instrument from the recognized at least one marker so as to obtain information of said at least one marker.

By "recognizing said at least one marker", it is understood that the at least one detected marker is analyzed so as to obtain information on the said at least one marker. The information of a marker is associated to characteristics of the surgical instruments and/or characteristics of the marker on the surgical instrument, which are already established for instance in an associative table. Recognizing a marker may allow obtaining the information of the position of the center of the marker in the captured frame; and/or the position of the center of each connected component associated together to create the marker. The code part may for instance correspond to a barcode from which a barcode value may be recognized. The barcode value allows obtaining information on said at least one marker. For instance, the barcode value of the marker may contain the marker orientation, the position on the surgical instrument and a surgical instrument identification number. In the case the extracted part of the frame corresponding to a marker requires compensating the deformation, an information on the angle of the marker in the captured frame referential (axes x from left to right on horizontal dimension and y from top to bottom on vertical dimension) is also available.

Preferably, the at least one part of the captured frame corresponds only to the code part of the detected marker.

Preferably, the computer-implemented method of the present invention may comprise a tracking method which may operate in real-time. The tracking method is performed on at least one marker, preferably on all the markers so as to improve the robustness of the tracking method. Also, the tracking method may be performed on at least one surgical instrument, preferably all the surgical instruments. Such tracking method can take into account all the information gathered from the previous computer-implemented method which have been performed.

The surgical instrument of the present invention may comprise multiple markers so as to improve the detection rate of the markers on the surgical instrument by the computer-implemented method of the present invention, by avoiding the possibility of an occlusion of every markers of a surgical instrument at once. For instance, when any surgical instrument is moved, a part of the surgical instrument may be hidden behind a tissue or an object such as another surgical instrument. Furthermore, using a plurality of markers enables minimizing the estimation error of the instrument tip. To this end, an estimation of the deformation associated to projection on the camera plane is performed, this estimation process being more robust as the number of markers is higher.

Advantageously, the step of identifying said at least one marker of a surgical instrument of the computer-implemented method is carried out using an artificial intelligence algorithm, the artificial intelligence algorithm operating a trained neural network, the neural network being trained for image processing with a deep learning algorithm during a training phase. Using a neural network allows ensuring robustness of the computer-implemented method according to the markers which are captured as deformed due to the projection on the camera plane. Using a neural network also allows network inference using graphics processing units features and allows maintaining a constant computation load.

In particular embodiments which may be combinable, the step of segmenting by colorimetry the at least one frame received of the computer-implemented method may be performed at least partially by an artificial intelligence algorithm which may be based on a convolution neural network applied onto the entire frame.

In particular embodiments, the computer-implemented method may be followed by a further method of performing image enhancement of the zoom on the computed area of interest so as to reconstruct better resolution frames.

Alternatively, the step of detecting within the at least one frame received at least one marker of the computer implemented method is carried out using an artificial intelligence algorithm, the artificial intelligence algorithm operating a neural network. Depending on the at least one frame received and on the pattern of the markers, the neural network is able to determine the best adapted steps for detecting said at least one marker. The neural network may for instance use segmentation by colorimetry so as to detect said at least one marker. Such neural network may be, among others, a convolution neural network or a recurrent neural network.

Preferably, the artificial intelligence algorithm used for carrying out the step of identifying said at least one marker of a surgical instrument is a convolution neural network comprising a convolution stage adapted for extracting the relevant features of the frames and a dense stage adapted for classifying the extracted features.

Advantageously, the artificial intelligence algorithm is a convolutional neural network. Preferably, the training of the convolution stage of the artificial intelligence algorithm uses an autoencoder to decode the extracted at least one marker from the frames and also to estimate the deformation and noise in this extracted at least one marker. Preferably, the extracted at least one marker corresponds only to the code part of the at least one marker. Preferably, the training of the dense stage is performed on the entire network with fixed and/or variable convolution layers weights. So as for the autoencoder to estimate the deformation and noise in the extracted markers, the autoencoder is trained by using as an input examples of extracted markers which may be deformed and/or present brightness irregularities and/or contrast issues and by using as an output the associated reference marker which is not deformed, which has no brightness or contrast irregularities. The associated reference marker corresponds to a reference image of a marker, i.e. an extracted marker image which is not deformed, does not contain noise or contrast irregularities. Therefore, the neural network convolution stage is trained so as to decode an extracted marker and predict a reference marker from such an extracted marker. Such deep learning database allows improving the skills of the artificial intelligence algorithm so as to reconstruct an extracted reference marker which is not deformed, which has no brightness or contrast irregularities. The loss function of the neural network is therefore minimized.

Advantageously, the deep learning phase comprises the steps of:

- training a convolution part of a convolutional neural network using an autoencoder; and

- training a dense part of a convolutional neural network with fixed and/or variable convolution layers weights.

The invention also discloses a learning database for training a neural network, in particular a convolutional neural network according to the previous paragraphs, the learning database comprising extracted markers and simulated markers generated from reference images on which are applied irregularities. Irregularities may refer to randomly applied deformations with a parabolic deformation profile and/or a brightness variation and/or a Gaussian noise.

By "extracted markers" it is understood markers which have been extracted from a captured frame, wherein these extracted markers may have been already identified so as to form a part of the learning database. By "simulated markers" it is understood markers generated from reference markers on which are applied different irregularities in the group of parabolic deformation, brightness variation, and Gaussian noise.

According to a second embodiment of the present invention, there is provided a learning database as further described in the appended independent claim 14.

The invention also discloses a system comprising:

- at least one surgical instrument according to the previous paragraphs;

- a processing unit configured for executing the computer-implemented method according to the previous paragraphs;

- a camera directed towards a surgical field and configured to send to the processing unit at least one frame; and

- a display screen configured to display the zoomed area of interest.

According to a third embodiment of the present invention, there is provided a system as further described in the appended independent claim 15.

Further characteristics, details and advantages of the invention will emerge from the description made with reference to the annexed drawings given by way of example.

BRIEF DESCRIPTION OF THE DRAWINGS

The invention will be better understood and its various characteristics and advantages will emerge from the following description of a number of exemplary embodiments and its appended figures.

Figure 1 displays a part of a frame captured by a camera corresponding to a surgical instrument wherein multiple markers are visible.

Figure 2 displays reconstructed markers from extracted markers from a frame captured by a camera.

Figure 3 displays a sample of multiple simulated markers of the learning database for training the convolutional neural network.

Figure 4 displays a flow chart of the computer-implemented method of the present invention in an embodiment.

DETAILED DESCRIPTION OF THE INVENTION

The invention discloses a surgical instrument having a tip end and comprising at least one marker, wherein said at least one marker is configured for identifying the surgical instrument, for indicating the position of said at least one marker on the surgical instrument and for indicating at least the orientation of the tip end of the surgical instrument.
Advantageously, the said at least one marker of the surgical instrument comprises a guide part and a code part, the guide part being configured to locate a marker, and wherein the guide part is of at least one color and the code part being of at least one other color which is different from the at least one color of the guide part.

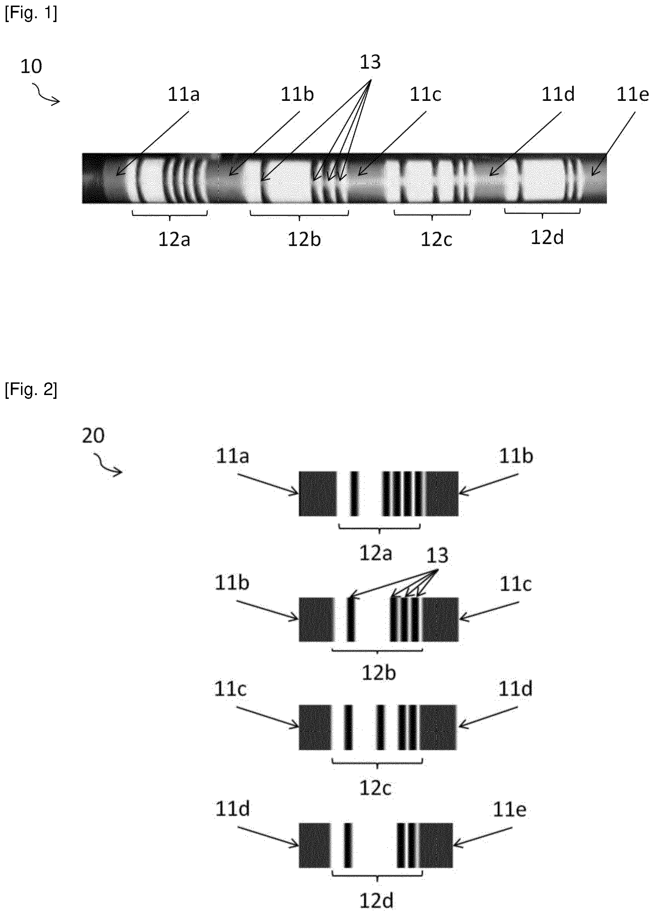

Figure 1 displays a part 10 of a frame captured by a camera corresponding to a part of a surgical instrument wherein several markers are visible. The visible part of the surgical instrument of Figure 1 corresponds to an elongated shape along an axis on which the markers are disposed. The part of the surgical instrument visible in Figure 1 comprises markers which are disposed as circumferential bands, orthogonally to the axis of the surgical instrument. In particular, four markers are visible wherein each marker is composed of two guide parts surrounding a code part. As such, the first marker comprises the guide parts 11a, 11b surrounding the code part 12a. The second marker comprises the guide parts 11b, He surrounding the code part 12b. The third marker comprises the guide parts 11c, 11d surrounding the code part 12c, and the fourth marker comprises the guide parts 11d, 11e surrounding the code part 12d.
The guide parts, generally denoted 11, correspond to circumferential bands of one color. The code parts, generally denoted 12, correspond to circumferential bands of two colors, being herein black and white. In this example, black circumferential bands, generally denoted 13 (indicated for only one marker on Figure 1) correspond to the information means for each code part. According to figure 1, some guide parts 11 of the several markers are confounded. Indeed, guide part 11b corresponds to guide part of both the first and the second markers. Guide part 11c corresponds to guide part of both the second and the third markers. Guide part 11d corresponds to guide part of both the third and the fourth markers. It can be observed in figure 1 that the brightness of the surgical instrument in the part of the frame 10 is not equivalent on the entire surgical instrument. Indeed, due to the reflection of a light emitted, the closest part of the round surface of the surgical instrument to the camera reflects a light particularly noticeable on the code part 12c. Also, it can be observed in figure 1 that the shape of the surgical instrument causes a deformation over the code parts visible in the part of the frame 10 captured. Indeed, as can be noticeably seen for the code part 12a the round shape of the surgical instrument causes the circumferential bands to appear as curved.
Alternatively, instead of surrounding the code part, the guide part may be positioned within the code part (not represented), the code part being placed on the two sides of a marker, a marker having two sides, one side being closer to the tip end of the surgical instrument, e.g. the distal end expected to interact with the patient tissue, and the other side being closer to the proximal end of the surgical instrument.
In another alternative, the at least one marker comprises a guide part and a code part which are composed of a plurality of sub-parts (not represented), the sub-parts of the guide part and the sub-parts of the code part being alternate. Other possibilities may be defined, the guide part and code part being intertwined or entangled in different manners which the skilled person may adapt for a particular use. In another particular embodiment, the guide part and the code part are confounded.
In particular embodiments, the guide part of a marker may be partially or completely confused with the guide part of another marker (not represented).
Advantageously, the guide part of the at least one marker of the surgical instrument is made of a contrasting color.

Preferably, the color of the guide part of the at least one marker of the surgical instrument is a contrasting color which is especially noticeable in an environment which is or is similar to human or animal tissues, in particular internal tissues. A contrasting color particularly noticeable in such environments may correspond to a visible light wavelength between 490 nm and 560 nm. Preferably, the color of the guide part of the at least one marker is green due to the low occurrence of the green color in such environments. However, the skilled person may adapt the choice of color for the guide part of the at least one marker of the surgical instrument according to the environment in which the surgical instrument is expected to be used.

Advantageously, the code part of the at least one marker of the surgical instrument includes a plurality of circumferential bands.

Advantageously, the at least one marker of the surgical instrument has a circumferential shape.

Also, the at least one marker may be an isolated sheet or an isolated tube which is particularly adapted to be provided onto a surgical instrument so as to obtain a surgical instrument of the present invention.

Advantageously, the surgical instrument further comprises a manipulating section at a first end, e.g. the proximal end, a tool section at a second end, e.g. the distal end, and an axis section between the manipulating section and the tool section, and wherein said at least one marker is located on the axis section.

In particular, the manipulating section of the surgical instrument (not represented) may correspond to a simple continuation of the axis section, for example a rod shape, as for instance in figure 1. The manipulating section may also have various shapes which are not to be considered as a limitation of the surgical instrument described herein. For example, the manipulating section may be gripped by a practitioner or a robot. The manipulating section may also comprise any type of mechanism which may operate the tool section.

The invention also discloses a computer-implemented method for determining the position and the orientation of the at least one tip end of a surgical instrument, the surgical instrument comprising at least one marker according to anyone of the previous paragraphs, the method comprising the steps of:

- receiving at least one frame captured by a camera;

- detecting within the at least one frame received at least one marker;

- identifying said at least one marker of a surgical instrument from the detected at least one marker so as to obtain information of said at least one marker; and

- determining the position and orientation of the at least one tip end of the surgical instrument using information of said at least one marker.

Advantageously, the computer-implemented method further comprises the steps of:

- computing an area of interest according to the determined position and/or orientation of the at least one tip end; and

- performing a zoom on the computed area of interest.

Advantageously, the step of detecting at least one marker in the computer-implemented method comprises:

- segmenting by colorimetry the at least one frame received;

- determining at least one part of a marker in the segmented frame; and

- detecting at least one marker associated to at least one determined part.

Advantageously, the step of identifying said at least one marker of a surgical instrument of the computer-implemented method comprises:

- extracting at least one part of the captured frame corresponding at least to the code part of said at least one detected marker and segmenting said at least one extracted part;

- recognizing said at least one marker from the segmented at least one extracted part; and

- identifying said at least one marker of a surgical instrument from the recognized at least one marker so as to obtain information of said at least one marker.

Figure 2 displays reconstructed markers 20 from extracted markers from a frame captured by a camera. Figure 2 corresponds to the result of an extraction and reconstruction of the markers in part 10 of a frame captured by a camera as shown on figure 1, corresponding to a part of a surgical instrument. The brightness of the surgical instrument in the part of the frame 10 of figure 1 is not equivalent on the entire surgical instrument and has been compensated in the reconstructed markers of figure 2. Also, it can be observed in figure 2 that the shape of the surgical instrument which caused a deformation over the code parts visible in the part of the frame 10 of figure 1 has been compensated so that the circumferential bands of the code parts do not appear as curved on the reconstructed markers.

Advantageously, the step of identifying said at least one marker of a surgical instrument of the computer-implemented method is carried out using an artificial intelligence algorithm, the artificial intelligence algorithm operating a trained neural network, the neural network being trained for image processing with a deep learning algorithm during a training phase.

Advantageously, the artificial intelligence algorithm is a convolutional neural network.

Advantageously, the deep learning phase comprises the steps of:

- training a convolution part of a convolutional neural network using an autoencoder; and
- training a dense part of a convolutional neural network with fixed and/or variable convolution layers weights.

The invention also discloses a learning database for training a neural network, in particular a convolutional neural network according to the previous paragraphs, the learning database comprising extracted markers and simulated markers generated from reference images on which are applied irregularities

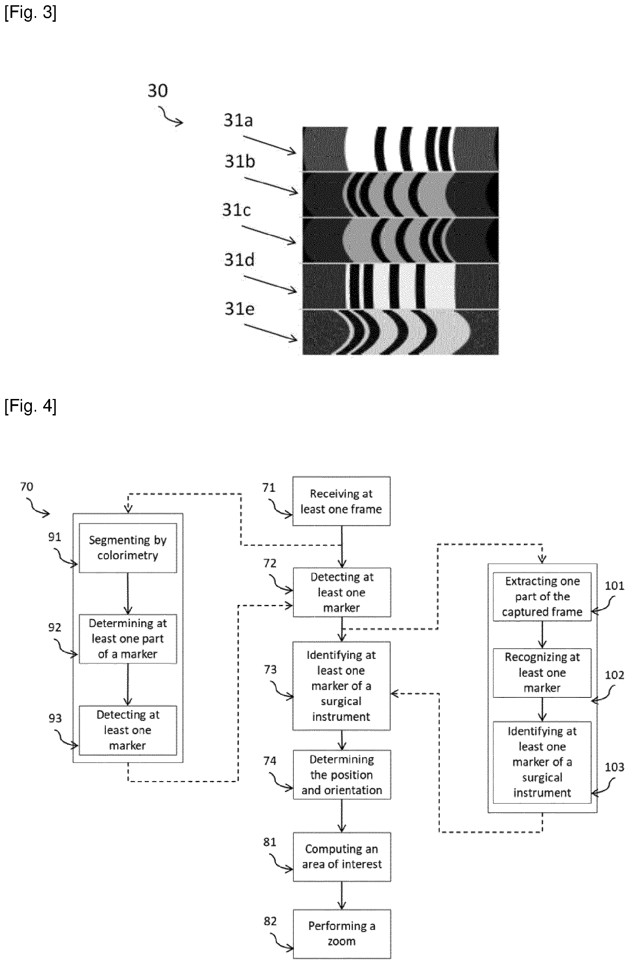

Figure 3 displays a sample 30 of a learning database for training a convolutional neural network. The exemplified sample 30 comprises simulated markers (31a to 31e) on which is applied randomly a deformation with a parabolic deformation profile and/or a brightness variation and/or a Gaussian noise. For instance, the simulated marker 31a presents a slight deformation; the simulated markers 31b and 31c present an important deformation and a decreased brightness, the simulated marker 31d presents a very slight deformation and the simulated marker 31e presents an important deformation and noises.

Figure 4 displays a flow chart 70 of an example of the computer-implemented method of the present invention. The computer-implemented method 70 comprises a step 71 of receiving at least one frame captured by a camera; a step 72 of detecting within the at least one frame received at least one marker; a step 73 of identifying at least one marker of a surgical instrument from the detected at least one marker so as to obtain information on said at least one marker; and a step 74 of determining the position and orientation of at least one tip end of the surgical instrument using information of said at least one marker; a step 81 of computing an area of interest according to the determined position and/or orientation of the at least one tip end; and a step 82 of performing a zoom on the computed area of interest.

The camera used according to the computer-implemented method may be any type of camera which is able to provide frames captured to a computer or a processing unit. For instance, the camera may be an endoscope 0°, 30°, 45°, e.g. a laparoscope or a thoracoscope, with a diameter of 5 or 10 mm and a variable direction of view 0-120°.

Optionally, step 72 may comprise a step 91 of segmenting by colorimetry the at least one frame received; a step 92 of determining at least one part of a marker in the segmented frame; and a step 93 of detecting at least one marker associated to at least one determined part.

The segmentation by colorimetry may comprise the transformation of the at least one frame received to at least one different color space such as for instance HSV and CIELab. In the case of multiple transformed frames, they may be combined as input by a AND operator so as to obtain an output binary frame. Thresholds of size of connected components may be set so as to avoid the noises, holes and separated connected components corresponding to a same marker. Morphological operations may then be performed on the at least one transformed frame, such as erosions to remove isolated pixels and dilatations to aggregate neighboring pixels.

Also optionally, step 73 may comprise a step 101 of extracting at least one part of the captured frame corresponding to said at least one detected marker and segmenting said at least one extracted part; a step 102 of recognizing at least one marker of a surgical instrument from the segmented at least one extracted part; and a step 103 of identifying said at least one marker of a surgical instrument from the recognized at least one marker so as to obtain information of said at least one marker.

In particular embodiments, wherein the surgical instrument comprises eight markers comprising a code part being a barcode, the code part of the markers can be recognized so as to obtain at least an identification number of each marker. The identification number of the marker recognized from a code part may correspond to a number between 0 and 7, 0 corresponding to the marker being the closest from the tip end of the surgical instrument and 7 corresponding to the farthest from the tip end. Each identification number of a marker has at least one associated characteristic which is encoded in an associative unit. The characteristics may be for instance the marker length, the code part length, the guide part length, the distance from the center of the marker to the tip end of the surgical instrument.

In particular embodiments combinable with the other embodiments, the code part of the markers can be recognized so as to obtain at least an identification number of a surgical instrument. The identification number of a surgical instrument may correspond to a number, for instance 1 or 2, corresponding to a single surgical instrument for number 1 and another surgical instrument for number 2. Each identification number of a surgical instrument has at least one associated characteristic which is encoded in an associative unit. The characteristics may be for instance the type of surgical instrument, the number of tip ends of the surgical instrument.

Therefore, in particular embodiments, the surgical instrument comprises multiple markers, each marker comprising a code part being a barcode. Each barcode may be recognized so as to obtain an identification number of a surgical instrument and an identification number of a marker. These two identification numbers for each marker allows obtaining the position on the marker on a surgical instrument which is identified.

In a particular embodiment, when at least one part of the captured frame corresponding to a detected marker is extracted, a deformation of the image, a variation of contrast or a brightness issue may appear. Indeed, as the surgical instrument may be orientated within different depth angles and because the surgical instruments are generally elongated and round, the extracted part of the marker may be difficult to recognize. Therefore, the method may comprise a sub-method of reconstructing the extracted part deformation so as to obtain an extracted undistorted part, i.e. a reconstructed part from the frame.

The extracted part of the captured frame, reconstructed or not according to the deformation, which corresponds to a marker may be processed by segmentation in as much classes as colors used in the at least one marker using a classical k-means method. From these classes a profile curve is constituted for each color by computing the number of pixels of each respective color along the extracted part of the frame. In the case of a marker comprising a guide part and a code part being made of circumferential bands, when the guide part surrounds the code part and when the code part is similar to a black and white barcode, profile curves of the colors of the parts may be obtained. Each profile curve is as long as the marker length. The profile curve of the guide parts, e.g. of green color, should demonstrate an amount of pixels at the beginning and the end of the curve. Complementarily, the profile curve of the colors of the code part should demonstrate an amount of pixels on different parts of the curve than the beginning and the end of the curve. A validation of the extracted frame may be done by analyzing the profile curves as respecting criteria of amount of pixels. The profile curve of the code part allows computing the information means and to obtain an extracted part of the frame which may be further recognized.

In certain embodiments, it is possible to identify a surgical instrument from the at least one marker recognized. For instance, the code part of two markers which are identified as being of the same identification number of surgical instrument are considered as being on one unique surgical instrument. Therefore, for such surgical instrument as an example, two markers which have been detected and identified as being on one unique surgical instrument allow determining the position and the orientation of the tip end of such surgical instrument by using the information obtained from the two markers.

By "identifying said at least one marker from the recognized at least one marker", it is understood in certain embodiments that the markers with the same identification number of a surgical instrument are associated together. However, the identification numbers of a surgical instrument obtained may correspond to a false alarm. In such a case, in order to associate only markers corresponding to the same surgical instrument, a logical hypothesis of the markers being aligned in the frame captured according to the shape of the instrument is considered. For instance, a distance between two markers can be computed so as to define if such distance is too high for considering that the two markers are aligned on the same surgical instrument. This may be achieved by using a polar coordinate system to describe markers position and orientation. For instance, the marker coordinate may be defined by $\theta$, being the angle between an x axis and the normal vector to marker axis, passing through the origin and R being the distance between marker axis and the origin. In such coordinate system, two markers which are aligned have positions close to each other. So a distance may be defined between a marker $m_1$ with coordinates $(r_1, \theta_1)$ and a marker $m_2$ with coordinates $(r_2, \theta_2)$ by using the following equation:

$$d(m_1, m_2) = \sqrt{\left(\frac{r_2 - r_1}{r_{max}}\right)^2 + \Delta\theta^2}$$

where $\Delta\theta$ is defined by:

$$\Delta\theta = \min(\theta_1 - \theta_2, \ \theta_1 - \theta_2 - 360, \theta_1 - \theta_2 + 360).$$

$r_{max}$ may be for example 200.

Therefore, the distance between a set of markers $m_{1\dots n}$ and another marker M is defined by:

$$d(M, m_{1\dots n}) = \min_{i=1\dots n}(d(m_i, M))$$

Then the markers association may be performed by using an algorithm in which $M_{i=1,n}$ is the list of markers to associate and $G_{i=1,m}$ is the list of groups of markers, corresponding to a single surgical instrument.

In particular embodiments, a requirement may be applied so that a group of markers with only one marker is not considered as a valid group of markers. Therefore, for a surgical instrument to be detected, at least two markers of such instrument should be visible and detected in the frame. Such constraint allows decreasing the false alarm rate, increasing the detection rate and moreover allows decreasing the error in the tip end position estimation process.

The invention also discloses a system comprising:

- at least one surgical instrument according to the previous paragraphs;

- a processing unit configured for executing the computer-implemented method according to the previous paragraphs;

- a camera directed towards a surgical field and configured to send to the processing unit at least one frame; and

- a display screen configured to display the zoomed area of interest.

**Claims**

1. A surgical instrument having a tip end and comprising at least one marker, wherein said at least one marker is configured for identifying the surgical instrument, for indicating the position of said at least one marker on the surgical instrument and for indicating at least the orientation of the tip end of the surgical instrument.

2. The surgical instrument as claimed in claim 1, wherein said at least one marker comprises a guide part (11) and a code part (12), the guide part being configured for locating a marker, and wherein the guide part is of at least one color and the code part being of at least one other color which is different from the at least one color of the guide part.

3. The surgical instrument as claimed in claim 2, wherein the guide part (11) is made of a contrasting color.

4. The surgical instrument as claimed in claims 2 or 3, wherein the code part (12) includes a plurality of circumferential bands (13).

5. The surgical instrument as claimed in anyone of the previous claims, wherein said at least one marker has a circumferential shape/rotational symmetry.

6. The surgical instrument as claimed in anyone of the previous claims, further comprising a manipulating section at a first end, a tool section at a second end, and an axis section between the manipulating section and the tool section, and wherein said at least one marker is located on the axis section.

7. A computer-implemented method (70) for determining the position and the orientation of at least one tip end of a surgical instrument, the surgical instrument comprising at least one marker according to anyone of claims 1 to 6, the method comprising the steps of:

   - receiving at least one frame captured by a camera (71);
   - detecting within the at least one frame received at least one marker (72);
   - identifying said at least one marker of a surgical instrument from the detected at least one marker (73) so as to obtain information of said at least one marker; and
   - determining the position and orientation of the at least one tip end of the surgical instrument using information of said at least one marker (74).

8. The computer-implemented method (70) as claimed in claim 7 further comprising the steps of:

   - computing an area of interest according to the determined position and/or orientation of the at least one tip end (81); and
- performing a zoom on the computed area of interest (82).

9. The computer-implemented method (70) as claimed in anyone of claims 7 to 8, wherein the step of detecting at least one marker comprises:

- segmenting by colorimetry the at least one frame received (91);
- determining at least one part of a marker in the segmented frame (92); and
- detecting at least one marker associated to at least one determined parts (93).

10. The computer-implemented method (70) as claimed in anyone of claims 7 to 9, wherein the step of identifying said at least one marker of a surgical instrument comprises:

- extracting at least one part of the captured frame corresponding at least to the code part of said at least one detected marker and segmenting said at least one extracted part (101);
- recognizing said at least one marker from the segmented at least one extracted part (102); and
- identifying said at least one marker of a surgical instrument from the recognized at least one marker (103) so as to obtain information of said at least one marker.

11. The computer-implemented method as claimed in anyone of claims 7 to 10, wherein the step of identifying said at least one marker of a surgical instrument is carried out using an artificial intelligence algorithm, the artificial intelligence algorithm operating a trained neural network, the neural network having being trained for image processing with a deep learning algorithm during a training phase.

12. The computer-implemented method as claimed in claim 11, wherein the neural network is based on a convolutional neural network.

13. The computer-implemented method as claimed in claims 11 or 12, wherein the training phase comprises the steps of:

- training a convolution part of a convolutional neural network using an autoencoder; and
- training a dense part of said convolutional neural network with fixed and/or variable convolution layers weights.

14. A learning database (30) for training a neural network as used in anyone of claims 11 to 13, the learning database comprising extracted markers and simulated markers (31) generated from reference images on which are applied different irregularities in the group of parabolic deformation, brightness variation, and Gaussian noise.

15. A system comprising:

- at least one surgical instrument according to anyone of claims 1 to 6;
- a processing unit configured for executing the method of anyone of claims 7 to 13;
- a camera directed towards a surgical field and configured to send to the processing unit at least one frame; and
- a display screen configured to display the zoomed area of interest.

[Fig. 1]

[Fig. 2]

[Fig. 3]

30

31a

31b

31c

31d

31e

[Fig. 4]

70

71 Receiving at least one frame

91 Segmenting by colorimetry

92 Determining at least one part of a marker

93 Detecting at least one marker

72 Detecting at least one marker

73 Identifying at least one marker of a surgical instrument

74 Determining the position and orientation

81 Computing an area of interest

82 Performing a zoom

101 Extracting one part of the captured frame

102 Recognizing at least one marker

103 Identifying at least one marker of a surgical instrument

Europäisches
Patentamt

European
Patent Office

Office européen
des brevets

## EUROPEAN SEARCH REPORT

**Application Number**

EP 20 30 5281

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 9 597 009 B2 (NOVARTIS AG [CH]) 21 March 2017 (2017-03-21) * column 3, line 62 - column 4, line 15; figures 1-3 * | 1-15 | INV. A61B90/00 G06N3/02 |
| X | EP 2 391 289 A1 (INTUITIVE SURGICAL OPERATIONS [US]) 7 December 2011 (2011-12-07) * paragraph [0047]; figure 22 * | 1 | |
| X | US 2009/171196 A1 (OLSON ERIC S [US] ET AL) 2 July 2009 (2009-07-02) * paragraph [0020]; figure 2 * | 1 | |
| X | WO 2013/126659 A1 (VERAN MEDICAL TECHNOLOGIES INC [US]) 29 August 2013 (2013-08-29) * paragraph [0165]; figure 4 * | 1 | |
| A | WO 2017/054817 A1 (CHRISTIANSEN OLAF [DE]) 6 April 2017 (2017-04-06) * the whole document * | 1-15 | TECHNICAL FIELDS SEARCHED (IPC) A61B G06N |
| A | US 2019/008412 A1 (DAVIES HELEN [GB]) 10 January 2019 (2019-01-10) * the whole document * | 1-15 | |
| A | WO 2018/218175 A1 (APPLIED MED RESOURCES [US]) 29 November 2018 (2018-11-29) * the whole document * | 1-15 | |

The present search report has been drawn up for all claims

| Place of search | Date of completion of the search | Examiner |
|---|---|---|
| Munich | 25 September 2020 | Held, Günter |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 20 30 5281

This annex lists the patent family members relating to the patent documents cited in the above-mentioned European search report.
The members are as contained in the European Patent Office EDP file on
The European Patent Office is in no way liable for these particulars which are merely given for the purpose of information.

25-09-2020

| Patent document cited in search report | | Publication date | Patent family member(s) | | Publication date |
|---|---|---|---|---|---|
| US 9597009 | B2 | 21-03-2017 | AU | 2014366911 A1 | 07-07-2016 |
| | | | CA | 2932895 A1 | 25-06-2015 |
| | | | CN | 105828703 A | 03-08-2016 |
| | | | EP | 3082569 A1 | 26-10-2016 |
| | | | ES | 2726895 T3 | 10-10-2019 |
| | | | JP | 6302070 B2 | 28-03-2018 |
| | | | JP | 2017501802 A | 19-01-2017 |
| | | | US | 2015173644 A1 | 25-06-2015 |
| | | | WO | 2015094726 A1 | 25-06-2015 |
| EP 2391289 | A1 | 07-12-2011 | CN | 102341054 A | 01-02-2012 |
| | | | EP | 2391289 A1 | 07-12-2011 |
| | | | KR | 20110118640 A | 31-10-2011 |
| | | | US | 2010168763 A1 | 01-07-2010 |
| | | | US | 2018071033 A1 | 15-03-2018 |
| | | | WO | 2010078016 A1 | 08-07-2010 |
| US 2009171196 | A1 | 02-07-2009 | US | 2009171196 A1 | 02-07-2009 |
| | | | WO | 2009085807 A1 | 09-07-2009 |
| WO 2013126659 | A1 | 29-08-2013 | EP | 2816966 A1 | 31-12-2014 |
| | | | US | 2013223702 A1 | 29-08-2013 |
| | | | US | 2013225942 A1 | 29-08-2013 |
| | | | US | 2013225943 A1 | 29-08-2013 |
| | | | US | 2013231556 A1 | 05-09-2013 |
| | | | US | 2015379710 A1 | 31-12-2015 |
| | | | US | 2017236272 A1 | 17-08-2017 |
| | | | US | 2019213735 A1 | 11-07-2019 |
| | | | US | 2019220976 A1 | 18-07-2019 |
| | | | WO | 2013126659 A1 | 29-08-2013 |
| WO 2017054817 | A1 | 06-04-2017 | DE | 112016004461 A5 | 21-06-2018 |
| | | | WO | 2017054817 A1 | 06-04-2017 |
| US 2019008412 | A1 | 10-01-2019 | EP | 3307136 A1 | 18-04-2018 |
| | | | US | 2019008412 A1 | 10-01-2019 |
| | | | WO | 2016185180 A1 | 24-11-2016 |
| WO 2018218175 | A1 | 29-11-2018 | NONE | | |

EPO FORM P0459

For more details about this annex : see Official Journal of the European Patent Office, No. 12/82